# EUROPEAN PATENT APPLICATION

(11) **EP 4 181 062 A1**
(43) Date of publication of application: **17.05.2023**
(21) Application number: 21207749.9
(22) Date of filing: 11.11.2021
(51) Int. Cl.: G06T 7/246

(54) **COMPUTER-ASSISTED MEDICAL PROCEDURES**

(71) Applicant: DENTSPLY SIRONA Inc., York, PA 17401 (US); Sirona Dental Systems GmbH, 64625 Bensheim (DE)
(72) Inventor: Kim, Hong Keun, 64625 Bensheim (DE)
(74) Representative: Özer, Alpdeniz

(57) **Abstract**

A method for improving a medical scan of head anatomy of a patient comprising: providing a plurality of scan datasets related to the head anatomy; providing intraoral data related to one or more intraoral features of the patient; determining, using the intraoral data, those parts of the scan datasets during acquisition of which a movement related to the intraoral features is detected; constructing and/or correcting the medical scan, from the scan datasets, in response to the determination. The present teachings also relate to a method of improving a CT scan, a system, a device, and a storage medium.

## Description

### TECHNICAL FIELD

The present teachings relate generally to computer-implemented methods and products for improving medical procedures.

### BACKGROUND

Certain kinds of medical procedures may require a patient to remain still until the procedure is completed. A movement of the patient in such situations may be detrimental to the quality of the procedure. For example, during medical imaging such as when capturing a computed tomography ("CT") scan it may be important for the patient to avoid movement in the anatomy which is being imaged. A movement in the anatomy may result in undesired artifacts which reduce the quality of the scan. Accordingly, unless the imaging operation is repeated to obtain a better scan, the health practitioner may be prevented from reaching a correct diagnosis. Similarly, in cases where the scan is to be interpreted using data-driven techniques, poor-quality scans may not be correctly interpreted by a data-driven logic. This may necessitate a repeat scan of the same anatomy, which increases chair-time for the patient. It may not only be inconvenient, but also result in higher radiation dose than necessary to the patient.

Similarly, in certain treatments, especially automated treatments, it may be required for the patient to suppress their movement such that the treatment may be applied at the correct location. For example, in procedures such as drilling at an intraoral location, patient movement may result in a poor result of the procedure.

The applicant has realized that there is a need of improved methods and products for assisting diagnosis and/or treatment procedures.

### SUMMARY

At least some of the limitations associated with the foregoing can be overcome by the subject matter of the accompanying independent claims. At least some of the additional advantageous alternatives will be outlined in the dependent claims.

The applicant has realized that at least some medical diagnosis and/or treatments involving anatomy of the head of a patient can be improved by more accurately determining patient movement, especially any movement occurring during the respective diagnosis and/or treatment. Especially in diagnosis and/or treatment which need to be performed in a serial fashion or in different sessions at different times, the present teachings can also allow aligning the patient's head anatomy at a desired position or a reference position even between different sessions. This can make the diagnosis and/or treatment more effective and/or comparable between sessions.

More specifically, the applicant has realized that patient head movement can be advantageously determined by tracking intraoral movement of the patient. Furthermore specifically, intraoral features such as dentition and/or gingiva and/or other intraoral anatomical features which are more rigidly attached to the skull, as compared to softer tissue such as tongue or cheeks, can be more advantageously tracked for determining head movement of the patient.

When viewed from a first perspective, there can be provided a computer-implemented method for improving a medical scan of head anatomy of a patient, which method comprises:
providing a plurality of scan datasets related to the head anatomy, wherein at least some of the scan datasets are usable for obtaining the medical scan;
providing intraoral data related to one or more intraoral features of the patient, which intraoral data having been captured during acquisition of the scan datasets;
determining, using the intraoral data, those parts of the scan datasets during acquisition of which a movement related to the intraoral features is detected;
constructing and/or correcting the medical scan, from the scan datasets, in response to the determination.

The applicant has realized that by doing so the medical scan can be improved by building the scan without at least some of those of the scan datasets, or parts thereof, during acquisition of which a movement related to the intraoral features is detected. In other words, the scan data which are corresponding to the movement are not included for constructing or reconstructing the medical scan, or said data are minimized while building the medical scan. Scan quality can thus be improved.

Additionally, or alternatively, in some cases, the intraoral data may even be used to determine a part of the scan dataset during acquisition of which a movement related to the intraoral features is detected, or even determine those scan datasets between acquisition of which a movement is detected. This can be beneficial to determine if patient alignment or position (e.g., position of the head anatomy) has changed between two acquisitions. The health practitioner can thus be alerted, and/or the effect of alignment or position corrected and/or compensated at least partially automatically in the medical scan, and/or the medical scan is constructed such as to remove or reduce the effect of the changed position.

The scan datasets associated with head movement, or motion associated datasets, can thus be rejected, replaced or reduced during building or reconstruction of the medical scan, and/or the effect of the motion associated datasets can be reduced or eliminated from the medical scan by applying a correction to an uncorrected medical scan which has been built including the motion associated scan dataset.

Accordingly, in such cases, the method may also comprise: constructing an uncorrected medical scan using the scan datasets.

"Medical scan" may refer an output of any kind of diagnosis scan performed on a patient, more particularly an extraoral scan. As a few non-limiting examples, the medical scan may result from a diagnosis or scanning procedure, such as an X-ray computed tomography ("CT"), magnetic resonance imaging ("MRI"), Single Photon Emission Computed Tomography ("SPECT"), positron emission tomography ("PET"), Heidelberg retina tomography ("HRT"), optical coherence tomography ("OCT"), ultrasound tomography, cone beam computer tomography ("CBCT"), for example dental CBCT and/or dental MRI or any combinations or variations of tomography imaging techniques as e.g. PET-CT or angiography. The medical scan may be in a human interpretable format and/or it may be in a computer readable format. For example, the medical scan may be in the form of a plurality of CBCT images. In some cases, the medical scan may even in the form of a digital three-dimensional ("3D") model of the patient's head anatomy.

By "constructing" the medical scan it is meant any kind of reconstruction or building of the medical scan from the scan datasets. For example, the medical scan may be a dental panoramic CBCT scan built or reconstructed from scan datasets in the form of a plurality of CBCT projection images captured via an X-ray source and a detector.

By "correcting" the medical scan it is meant any kind of operation performed on an at least partially uncorrected medical scan to remove one of more undesired effects or artifacts arising due to head movement of the patient during acquisition of the scan datasets. Thus, correcting may refer to performing any one or more operations for reducing the effect of the movement from the medical scan, including: deleting, replacing, averaging, compensating, recalculating, transforming, extrapolating, or masking the motion associated scan datasets, or effect thereof, in an at least partially uncorrected medical scan.

Thus, according to an aspect, the correcting and/or constructing of the medical scan involves performing any one or more operations for reducing the effect of the movement from the medical scan, more particularly the operations including any one or more of: deleting, replacing, averaging, compensating, transforming, recalculating, extrapolating, or masking, data of the parts of the scan datasets. Thus, any one or more of the operations or processing functions such as including, discarding, selecting, applying a weighting factor, deleting, replacing, averaging, compensating, transforming, recalculating, extrapolating, or masking may be applied to any one or more of the scan datasets. The one or more of the processing functions may be applied in response to the determination.

Additionally, or alternatively, the correcting of the medical scan may involve transforming coordinates from the intraoral data to coordinates related to the scan datasets. Accordingly, by applying at least one coordinate transform, at least a two-dimensional movement, preferably a three-dimensional movement, can be used for correcting of the medical scan. This can allow the movement to be corrected along the relevant coordinates. Moreover, a knowledge of the direction of the movement can allow a more suitable correction to be applied. Any suitable transformation technique may be applied to determine the movement from the intraoral data and transform the same to the coordinate system of the scan data, for example techniques usable in the field of computer vision.

Hence, according to an aspect, the method comprises: applying a relative coordinate transformation determined from the intraoral data to a coordinate system of the medical scan.

For example, the transformation may involve applying a change of position and/or orientation of one or more features from the intraoral data for calculating a corresponding change in the coordinate system of the scan data.

More preferably, the movement is provided a measurement value in the intraoral data. An advantage of doing so can be that a quantified measure of correction may be applied dependent upon the measure of the movement, thus resulting in a better correction of the medical scan.

According to an aspect, an Iterative Closest Point ("ICP") transform, their likes, or any variant thereof, is used for coordinate transformation between the intraoral data and the corresponding scan datasets.

The relative coordinate transformation converts a movement measure, of the movement, which is calculated from the intraoral data, to one or more movement correction values for the medical scan and/or the corresponding data during which the movement was measured. Thus, the transformation can be used for correcting the medical scan.

By "head anatomy" it is meant any part of the head of a patient. In the present context, it is meant those anatomical parts which are to be diagnosed and/or treated. For example, the head anatomy may refer to any cranial part of the patient. In addition, or alternatively, the head anatomy may refer to mandibular anatomy. As further non-limiting examples, the head anatomy may refer to any one or more of dentition, jaw, gingiva, tongue, bone, nerves, eye, brain, skull, and blood vessels of the head.

The term "scan datasets" may refer to data which are acquired from a diagnosis or scanning procedure performed on the head anatomy. The data may be captured continuously, sequentially, or intermittently with regular or irregular time periods. At least some of the scan datasets may each individually be a self-contained dataset, for example a scan dataset may be in the form of a single image captured during the diagnosis or scanning procedure. In some cases, a scan dataset may be in the form of a plurality of images. For example, the scan data may be in the form of point cloud data, grid, mesh, or their likes. Alternatively, or in addition, each dataset may comprise one or more values of predetermined one or more parameters measured during the diagnosis or scanning procedure. Alternatively, or in addition, at least some of the scan datasets may be parts of a data block or data stream generated during the diagnosis or scanning procedure. The data block or data stream may comprise a plurality of images and/or measured values of predetermined parameters. More particularly, the scan datasets refer to those data which are generated from an extraoral diagnosis or scanning procedure. The scan datasets may also comprise corresponding timestamps at which each dataset or perhaps even each datapoint within the dataset is generated. Accordingly, the scan data or datasets may be in the form of time-series data. The scan datasets may be provided at a memory location or data storage, e.g., a database.

"Intraoral features" refers to information related to intraoral structures such as dentition and/or gingiva and/or other intraoral anatomical features which are more rigidly attached to the skull. Alternatively, or in addition, at least some of the intraoral structures of which intraoral features are monitored or tracked may be one or more artificial structures such as one or more dental replacements, e.g., dental crown, braces, veneer, bridge. Alternatively, or in addition, the intraoral structure may in some cases even be a scan body (or scanbody) or other natural and/or artificial structure similarly rigidly attached to the jaw of the patient.

More particularly, the information related to intraoral structures which is tracked via the intraoral features may be any one or more of: position, displacement, distance, velocity, or any other parameter which can indicate a movement in said structure. The movement may be measured with respect to a reference, e.g., with respect to a predetermined point on earth or with reference to a point or location fixed with respect to a predetermined point on earth. Hence, in some cases, the movement may be measured relatively respect to another point or location. Preferably, the intraoral features are measured or monitored via optical measurements of said features, for example, using a camera, scanner or any other sensor which is usable for detecting movements of intraoral features. More particularly, the intraoral features are measured or monitored via at least one sensor in an intraoral part. The intraoral part may comprise an intraoral portion which is positionable in the oral cavity of the patient prior to acquiring the plurality of scan datasets. Any one or more of the sensors may be included in the intraoral portion, and/or any one or more of the sensors may be located external of the intraoral portion which is positionable in the oral cavity. The external sensors may be functionally coupled via a suitable channel with the intraoral portion such that the external sensors are able to detect movement of the intraoral features when the intraoral portion which is positioned in the oral cavity. The channel may be, or it may comprise, for example, any one or more of an optical channel, a prism, a mirror, a beam deflector, an optical splitter, a lens, or any other optical element. In some cases, the intraoral part may also include one or more light sources, located in the intraoral portion and/or external of the intraoral portion. The light sources may be used to illuminate the intraoral anatomy for assisting the sensors to detect movement of the intraoral features. Similar to the external sensors, the external sensors may be functionally coupled via a suitable channel, such as an optical channel, with the intraoral portion. The intraoral part will be discussed in additional detail later.

A "movement" related to the intraoral features may be measured by any suitable parameter which indicates a movement of the intraoral features with respect to a reference point or relative to the reference point. For example, the movement may be measured by measuring a distance, an orientation, or displacement of any one or more of the intraoral features. Additionally, or alternatively other suitable parameter may be used. For example, speed or velocity, acceleration, rotation, etc. The reference point may be a point fixed on any place on earth or it may be a relative reference with respect to which the parameter is measured.

"Intraoral data" refer to one or more signals and/or data which comprise information related to one or more intraoral features. The information may be in the form of any measurable parameter related to the intraoral features or structure which can indicate a movement of said structure. The intraoral data may be generated via one or more sensors which are disposed for monitoring the intraoral features. The intraoral data are acquired simultaneously to the acquisition of the scan data or datasets. For example, the intraoral data may be in the form of surface data, point cloud, grid, mesh, or their likes. The intraoral data may even comprise timestamps. Accordingly, in some cases, the intraoral data may be in the form of time-series data.

Preferably, the intraoral data are at least partially generated via a sensor suitable for detecting movements in intraoral features during the diagnosis or scanning procedure. In some cases, the intraoral data may be in the form of a data stream generated during the acquisition of the scan data or datasets. In some cases, the intraoral data may only be generated in an event when a movement is detected. Such event based intraoral data may either directly be linked to the corresponding part of the scan data or one or more scan datasets which may be affected by the movement, and/or the event based intraoral data may be provided with timestamps which are then used to for constructing and/or correcting the medical scan, e.g., by correlating or finding the part of the scan data or one or more scan datasets which are associated with the time at which the movement occurred. Preferably, the intraoral data comprise at least one measured value of the movement. The measured value may be a quantification of how large the movement has been, in a 2D or 3D space. Additionally, the measured value may also comprise, or it is provided with a directional information. The directional information may be indicative of the direction in which the movement occurred.

According to an aspect, in response to detection of a movement in the intraoral features, the corresponding scan datasets or parts thereof are marked or tagged. This is preferably done while the diagnosis scan is being performed. An advantage of doing so can be that the health practitioner or technician performing the scan can be alerted specifically about the parts of the diagnostic scan where movement artifacts may arise. The practitioner may thus be enabled to take a corrective action, e.g., by repeating the scan of the spot during scanning of which the movement occurred.

Alternatively, or in addition, as mentioned, the timestamps of the intraoral data may be provided in a time-synchronized state with the timestamps of the scan datasets such that the timestamps or a range thereof during which a movement is detected are recorded. The corresponding scan datasets or parts thereof which may be affected by the movement can thus even be determined post acquisition of the scan datasets.

In some cases, a reference intraoral position may be determined for detecting the movement. Thus, according to an aspect, the method may also comprise: determining one or more reference intraoral positions with respect to one or more of the intraoral features.

The reference positions of one or more corresponding intraoral features may be used for detecting if a specific feature has moved during the capturing of the scan data. The reference positions may even be used for quantifying the movement or for measuring the magnitude and/or direction of the movement. This can make it easy to track any movement from a desired reference position. A reference position in context may refer to a position with respect to which a movement is detected. For example, an initialization step may be performed at the start which records an initial position of certain intraoral features at that time. Then, as the scan datasets are acquired determination may be made if the position of any of these features has changed with respect to the initial or reference position.

The method may also comprise: determining one or more movement values of the one or more of the intraoral features from their respective reference intraoral positions.

The movement value or measure may, for example, be determined by measuring displacement of the intraoral features from their respective reference intraoral positions. Additionally, or alternatively, any one or more other movement measures such as velocity and acceleration can be determined.

According to a more specific aspect, e.g., for tomography applications of the present teachings, there can be provided a computer-implemented method for improving a computer tomography scan of cranial and/or mandibular anatomy of a patient, which method comprises:
providing a plurality of tomographic images of the cranial and/or mandibular anatomy, wherein at least some of the tomographic images are usable for obtaining the computer tomography scan;
providing intraoral data related to one or more intraoral features, which intraoral data having been captured during acquisition of the tomographic images;
determining, using the intraoral data, those of the tomographic images during acquisition of which a movement related to the intraoral features is detected;
constructing and/or correcting the computer tomography scan based upon the determination.

It shall be appreciated that the "tomographic images" may be in the form of one or more datasets.

Related to the aforementioned, those skilled in the art shall appreciate that when viewed from another perspective, the present teachings can also provide a computer-implemented method for improving a medical scan of head anatomy of a patient, which method comprises:
providing an intraoral part deterministically positioned relative to an acquisition part;
acquiring, via the acquisition part, the plurality of scan datasets of the head anatomy;
capturing during acquisition of the scan datasets, via the intraoral part, intraoral data related to one or more intraoral features;
determining, using the intraoral data, those of the scan datasets during acquisition of which a movement related to the intraoral features is detected;
constructing and/or correcting the medical scan based upon the determination.

It shall be appreciated that the medical scan is constructable from the plurality of images acquired via the acquisition part.

"Intraoral part" refers to a part via which intraoral data are captured. As it was discussed previously, the intraoral part may comprise an intraoral portion which is positionable in the oral cavity of the patient. The intraoral part may comprise one or more sensors and optionally one or more light sources. The sensors and/or light sources may be located in the intraoral portion or external of the intraoral portion. Thus, in some cases the intraoral part may comprise the intraoral portion and an extraoral portion connected to the intraoral portion. At least some of the light sources and/or sensors may thus be located in the extraoral portion. The extraoral portion is thus that part of the intraoral part which is not required to be inserted in the patient's mouth. For example, the intraoral portion may be a bite block which is inserted in the patient's mouth. The bite block may be connected to an extraoral portion. The bite block may optionally comprise optical means such as one or more lenses for measuring intraoral features. The bite block may optionally comprise further optical means for projecting light on the intraoral features. The optical means and the further optical means may be the same optics or different optics. In cases where the light source and/or sensor are located outside of the intraoral portion, the corresponding optics may be coupled to one or more optical channels for communicating the corresponding signal from and/or to the external light source and/or sensor. Thus, for optical measurements the light source and/or sensor are optically coupled either directly with the optics in the intraoral portion or via the optical channel connecting the optics and the extraorally located light source and/or sensor.

The bite block may be disposable, or it may be reusable, for example, after sanitizing.

An advantage of placing sensors and light sources in the extraoral portion rather than in the intraoral portion can be that the intraoral portion can be made of material which does not interfere with the medical scan. For example, for diagnosis involving X-ray, metallic material may interfere with the scan and result in artifacts. Thus, by avoiding electronics within the intraoral portion, intraoral data can be reliably recorded without interfering or affecting the quality of the diagnosis operation.

As discussed, the intraoral part, or more specifically, the intraoral portion, may be in the form of a bite block. This can have an advantage that the intraoral features are aligned better for recording the intraoral data.

In some cases, the intraoral part may be in the form of, or it may comprise, a dental scanner. For example, a handheld dental scanner can be adapted to be used for providing the intraoral data. For example, the dental scanner may be provided a mount which allows it to be deterministically positioned relative to the acquisition part. For example, the dental scanner may be held fixedly in the mount, which is attached to an object such as the body of the diagnosis and/or treatment system, for acquiring the intraoral data. The scanner may be permanently fixed with the mount or it may be releasably attachable with the mount. An advantage of doing so can be that the intraoral scanner can be leveraged both for acquiring the intraoral data as well being usable in a normal manner as a dental scanner. It shall be appreciated that the portion of the scanner which is insertable in the patient's oral anatomy may be considered as the intraoral portion, while the portion which remains outside may be considered the extraoral portion. According to an aspect, the dental scanner may be provided with an endpiece attachable on the sensor side of the scanner. The endpiece may attach over the scanner, or it may replace the usual endpiece used for dental scanning. For example, the endpiece may comprise the biteblock. After the scanner is used for acquiring intraoral data, the scanner may be removed from the endpiece and the usual endpiece may be attached for conventional use, e.g., as a dental scanner for generating 3D model of the oral anatomy. In some cases, the endpiece may comprise the channel which couples the intraoral portion (e.g., biteblock or bite block) with the extraoral portion (e.g., handheld portion of the scanner). As it was discussed previously, it can also be envisaged to have a movable mount allows the scanner or the intraoral portion thereof to be deterministically positioned relative to an acquisition part. The endpiece may or may not be fully integrated with the biteblock. The endpiece and/or the biteblock may be disposable or it may be reusable. In some cases, the endpiece and/or the biteblock may be specially manufactured to prevent fogging of any optical elements which are used for monitoring the intraoral features. This may include any one or more of: providing a heating element for warming the optical element for preventing fogging, providing the optical element with a non-fogging film. Any reusable parts may be sanitizable, e.g., by heating and/or washing e.g., with disinfectant. Any specifics of such components are not limiting to the scope or generality of the present teachings.

"Acquisition part" refers to part of the treatment and/or diagnosis system via which the scan datasets are acquired. For example, the acquisition part may be an X-ray sensor. In some cases, the acquisition part may even refer to the X-ray sensor and the corresponding X-ray source.

The intraoral part is deterministically positioned relative to the acquisition part. It means that at any time, position of the intraoral part can be determined vis-à-vis position of the acquisition part. Hence, at any time, by knowing coordinates related to the intraoral part, coordinates related to the acquisition part can be determined, or *vice versa.* In some cases, the intraoral part may be rigidly attached to a fixed object, for example, the body or frame of a machine which is used for performing the diagnosis and/or treatment. In such a case, if a movement is detected in the patient's intraoral features, the corresponding movement can be calculated for the scan for the purpose of correction. Alternatively, the movement detection may just be used for eliminating or reducing the contribution of the corresponding scan dataset from the medical scan. Those skilled in the art shall appreciate though that even if the intraoral part is rigidly attached to a fixed object, the acquisition part may or may not be. Hence, the acquisition part may be stationary or movable with respect to the intraoral part. In some cases, both the acquisition part and the intraoral part or the intraoral portion may be movable with respect to each another. They may still be deterministically positioned relative to each another, for example, via measurements of the respective movements of the parts.

Thus, it shall be appreciated that the movement related to the intraoral features may be detected by measuring movement between the intraoral part or portion and the corresponding oral anatomy of the patient, or it may be detected by measuring the movement of the intraoral part or portion. The former situation can occur when the intraoral part or portion is fixedly or relatively rigidly attached to an object, while the latter case can correspond to when the intraoral part or portion is held tightly in the patient's mouth, but the part of portion is not fixedly attached to an object. Thus, in this latter case, if the patient moves their head, the intraoral part of portion moves accordingly. By measuring the movement of the intraoral part or portion, the movement of the head can be calculated. The movement measure can then be used for correcting and/or constructing the medical scan.

When viewed from yet another perspective, there can also be provided a system comprising means for performing any of the herein disclosed methods. Or there can be provided a system, wherein the system is configured to perform any of the herein disclosed methods.

For example, there can be provided medical scanning system comprising:
an active part for performing the medical scan;
an intraoral part positioned deterministically relative to the active part; the intraoral part comprising: an intraoral portion which is positionable in the oral cavity of the patient prior to acquiring a plurality of scan datasets; and a sensor for monitoring one or more intraoral features of the patient and generating intraoral data including said features during acquisition of the tomographic images,
a computing unit configured to:
   determine, using the intraoral data, those of the scan datasets during acquisition of which movement related to the intraoral features is detected; and
   construct and/or correct medical scan based upon the determination.

As it was discussed, the medical scan is constructed from the scan datasets.

Or, more generally, when viewed from another perspective, the present teachings can also provide a system comprising:
an active part for performing treatment and/or diagnosis of a patient;
an intraoral part deterministically positionable relative to the active part; the intraoral part comprising: an intraoral portion which is positionable in oral cavity of the patient prior to performing the treatment and/or diagnosis; and, a sensor for: monitoring one or more intraoral features of the patient; and, generating intraoral data including said features during the treatment or diagnosis,
a computing unit configured to:
   determine, from the intraoral data, a movement related to the intraoral features; and
   adapt the treatment and/or diagnosis in response to the movement.

Those skilled in the art shall appreciate that by saying here "a sensor", it is also meant to include "at least one sensor".

Especially in cases where the diagnosis and/or treatment relates to a subsequent session, there can also be provided a system comprising:
an active part for performing treatment and/or diagnosis of a patient;
an intraoral part deterministically positionable relative to the active part; the intraoral part comprising: an intraoral portion which is positionable in oral cavity of the patient prior to performing the treatment and/or diagnosis; and, a sensor for: monitoring one or more intraoral features of the patient; and, generating intraoral data including said features,
a computing unit configured to:
   determine, from the intraoral data, a movement and/or position related to the intraoral features; and
   adapt the treatment and/or diagnosis in response to the movement.

Thus, in cases where patient position or more specifically the patient's head anatomy position is to be aligned for different sessions, the intraoral data may also comprise data from a previous treatment and/or diagnosis session. The previous intraoral data, in such cases, comprised in the intraoral data may for example be in the form of reference position data which are usable for aligning the intraoral features at the same or similar position relative to the intraoral part, as in the previous session. This can have an advantage that by more accurately aligning the patient, more comparable diagnosis and/or treatment can be provided to the patient.

By "adapting" the treatment and/or diagnosis it is meant correcting and/or constructing a medical scan in response to the movement as it was discussed above. Additionally, or alternatively, adapting the treatment and/or diagnosis may comprise reducing or eliminating effect of the movement on the treatment. For example, the treatment may be a surgical procedure being performed on the patient, thus by detecting the movement, the active part may be adjusted, paused, realigned, or in general adapted such that any undesired effect of the movement of the treatment procedure is reduced or eliminated. Hence, a more accurate and robust positioning of diagnosis and/or treatment instrument can be enabled. This can be particularly advantageous in automated or robotic treatments by making such treatments safer from being affected by patient head movements. In some procedures, the patient head is presently required to be immobilized. The present teachings at least in some of such cases prevent a need of at least total head immobilization by instead adapting the treatment itself in response to the movement. Optionally, in some cases, movement of other body parts of the patient may also be tracked via one or more extraoral sensors. Thus, in general the present teachings can allow a real-time tracking or monitoring of head movements using intraoral data. Advantageously, such tracking or monitoring can be used to adapt the diagnosis and/or treatment.

By "treatment" it is here meant any kind of physical, mechanical, thermal, particle, or radiation-based treatment. Some non-limiting examples where the present teachings can be applied include tumor therapy, radiation therapy, stereotactic radiosurgery, proton therapy, heavy ion therapy, and dental therapies. As a non-limiting example, the treatment may be provided to the patient via a medical robot or robotic arm, either fully autonomous, partially autonomous, or controlled via a dental practitioner. Another non-limiting example for medical treatment is radiation or particle-based tumor therapy.

Any of the systems herein disclosed may comprise one or more computing units. Optionally, any of the systems and/or any of the computing units may be configured to operatively connect to a network, e.g., via one or more network interfaces and/or connectivity interfaces.

The memory location may be a part of a computing unit and/or the system, or it may be located remotely operatively accessible via any of the computing units.

When viewed from another perspective, there can also be provided a computer software product, or a non-transitory computer-readable storage medium storing the program, comprising instructions which when executed by a suitable computing unit, or a system comprising the computing unit, cause the computing unit to perform any of the herein disclosed methods.

For example, there can be provided a computer software product, or a non-transitory computer-readable storage medium storing the program, comprising instructions which when executed by one or more computing units, cause any of the computing units to:
provide a plurality of scan datasets related to the head anatomy, wherein at least some of the scan datasets are usable for obtaining the medical scan;
provide intraoral data related to one or more intraoral features of the patient, which intraoral data having been captured during acquisition of the scan datasets;
determine, using the intraoral data, those parts of the scan datasets during acquisition of which a movement related to the intraoral features is detected;
construct and/or correct the medical scan, from the scan datasets, in response to the determination.

The computer software product, or a non-transitory computer-readable storage medium storing the program, may even comprise instructions which when executed by the suitable computing unit cause the computing unit to:
operatively connect to a memory location for obtaining or accessing the scan datasets and/or the intraoral data.

A computer-readable data medium or carrier includes any suitable data storage device on which one or more sets of instructions (or software) are stored for implementing any one or more methodologies disclosed herein. The instructions may even reside partially, or completely, within the main memory and/or within the processor during execution thereof by the processing unit, computing unit, and main memory, which may constitute computer-readable storage media. The instructions may even be transmitted or received over a network via a network device.

The computer program for implementing any one or more of the embodiments described herein may be stored and/or distributed on a suitable medium, such as an optical storage medium or a solid-state medium supplied together with or as a part of another hardware, but may also be distributed in other forms, such as via the Internet or other wired or wireless telecommunication systems. However, the computer program may also be presented over a network like the World Wide Web and can be downloaded into the working memory of a data processor from such a network.

Furthermore, data carrier or a data storage medium for making a computer program product available for downloading can also be provided, which computer program product is arranged to perform a method according to any of the methods herein disclosed.

When viewed from another perspective, there can also be provided a computing unit comprising the computer program code for performing the method herein disclosed. Also, there can be provided a computing unit operatively coupled to a memory storage comprising the computer program code for carrying out any of the methods herein disclosed.

That two or more components are "operatively" coupled or connected shall be clear to those skilled in the art. In a non-limiting manner, this means that there may be at least one communicative connection between the coupled or connected components e.g., they are the network interface or any suitable interface. The communicative connection may either be fixed, or it may be removable. Moreover, the communicative connection may either be unidirectional, or it may be bidirectional. Furthermore, the communicative connection may be wired and/or wireless. In some cases, the communicative connection may also be used for providing control signals.

"Computing unit" or "processing unit" may comprise, or it may be, a processing means or computer processor such as a microprocessor, microcontroller, or the likes, having one or more computer processing cores.

"Computer processor" refers to an arbitrary logic circuitry configured for performing basic operations of a computer or system, and/or, generally, to a device which is configured for performing calculations or logic operations. In particular, the processing means or computer processor may be configured for processing basic instructions that drive the computer or system. As an example, the processing means or computer processor may comprise at least one arithmetic logic unit ("ALU"), at least one floating point unit ("FPU"), such as a math coprocessor or a numeric coprocessor, a plurality of registers, specifically registers configured for supplying operands to the ALU and storing results of operations, and a memory, such as an L1 and L2 cache memory. In particular, the processing means or computer processor may be a multi core processor. Specifically, the processing means or computer processor may be or may comprise a central processing unit ("CPU"). the processing means or computer processor may be a complex instruction set computing ("CISC") microprocessor, reduced instruction set computing microprocessor ("RISC"), Very long instruction word ("VLIW") microprocessor, a processor implementing other instruction sets or processors implementing a combination of instruction sets. The processing means may also be one or more special purpose processing devices such as an application specific integrated circuit ("ASIC"), a field programmable gate array ("FPGA"), a complex programmable logic device ("CPLD"), a digital signal processor ("DSP"), a network processor, or the like. The methods, systems and devices disclosed herein may be implemented as software in a DSP, in a microcontroller, or in any other side processor such as hardware unit within an ASIC, CPLD, or FPGA. It is to be understood that the term processing means or processor may also refer to one or more processing devices, such as a distributed system of processing devices located across multiple computer systems (such as cloud computing), and is not limited to a single device unless otherwise specified.

"Connectivity interface" or "communication interface" refers to a software and/or hardware interface for establishing communication such as transfer or exchange of signals or data. The communication may either be wired, or it may be wireless. Connectivity interface is preferably based on or it supports one or more communication protocols. The communication protocol may be a wireless protocol, for example: short distance communication protocol such as Bluetooth^{®}, or Wi-Fi, or long communication protocols such as cellular or mobile network, for example, second generation cellular network ("2G"), 3G, 4G, long term evolution ("LTE"), or 5G. Alternatively, or in addition, the connectivity interface may even be based on proprietary short distance or long distance protocol. The connectivity interface may support any one or more standards and/or proprietary protocols.

"Network interface" refers to a device or a group of one or more hardware and/or software components that allow an operative connection with the network.

"Memory storage" may refer to a device for storage of information, in the form of data, in a suitable storage medium. Preferably, the memory storage is a digital storage suitable for storing the information in a digital form which is machine readable, for example digital data that are readable via a computer processor. The memory storage may thus be realized as a digital memory storage device that is readable by a computer processor. Further preferably, the memory storage on the digital memory storage device may also be manipulated by a computer processor. For example, any part of the data recorded on the digital memory storage device may be written and or erased and or overwritten, partially or wholly, with the new data by the computer processor.

"Network" discussed herein may be any suitable kind of data transmission medium, wired, wireless, or their combination. A specific kind of network is not limiting to the scope or generality of the present teachings. The network can hence refer to any suitable arbitrary interconnection between at least one communication end point to another communication end point. Network may comprise one or more distribution points, routers or other types of communication hardware. The interconnection of the network may be formed by means of physically hard wiring, optical and/or wireless radio frequency ("RF") methods. The network specifically may be, or it may comprise, a physical network fully or partially made by hard wiring, such as a fiber optical network or a network fully or partially made by electrically conductive cables or a combination thereof. The network may at least partially comprise the Internet.

### BRIEF DESCRIPTION OF THE SEVERAL VIEWS OF THE DRAWINGS

Certain aspects of the present teachings will now be discussed with reference to the accompanying drawings that explain said aspects by the way of examples. Since the generality of the present teachings is not dependent on it, the drawings may not be to scale. Method and system aspects may be discussed in conjunction for ease of understanding. Certain features shown in the drawings may be logical features that are shown together with physical features for the sake of understanding and without affecting the generality or scope of the present teachings.
FIG. 1 illustrates a CBCT system pursuant to the present teachings;
FIG. 2 illustrates an alternative CBCT system pursuant to the present teachings;
FIG. 3 illustrates another view of a CBCT system;
FIG. 4 illustrates a zoomed in view of the teachings when applied to the CBCT system;
FIG. 5 illustrates a bite block pursuant to an aspect of the present teachings;
FIG. 6 illustrates application of the present teachings to an alternative diagnosis/treatment system;
FIG. 7 illustrates an example of patient head immobilization;
FIG. 8 illustrates an aspect of the present teachings when applied to a diagnosis/treatment system;
FIG. 9 illustrates a flowchart 900 in accordance with an aspect;
FIG. 10 illustrates another flowchart 1000 in accordance with another aspect;
FIG. 11 illustrates a further flowchart 1100 in accordance with yet another aspect.

### DETAILED DESCRIPTION

In accordance with example aspects described herein, methods, systems and computer readable storage media can be provided, e.g., methods for improving a medical scan of head anatomy of a patient.

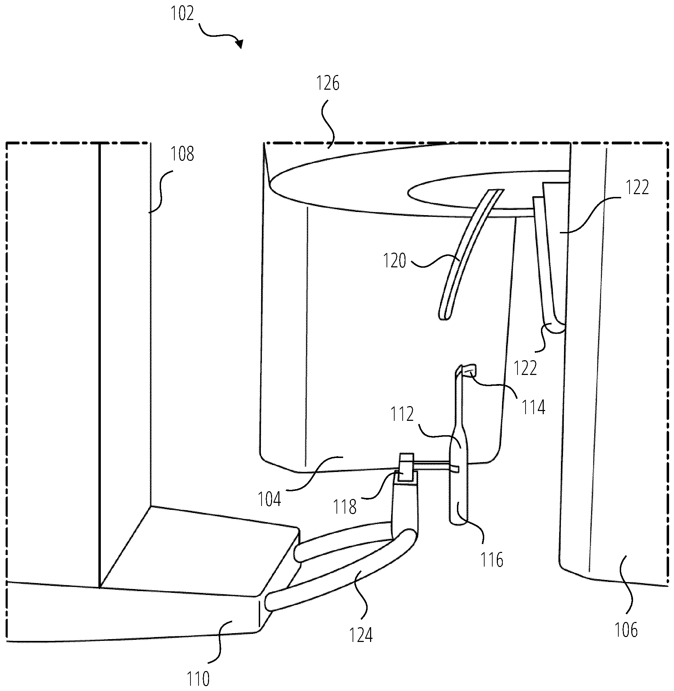

FIG. 1 illustrates a diagnosis system 102 shown in this example as a CBCT machine 102. Cone beam computed tomography or CBCT is used for obtaining a medical scan. The CBCT machine 102 comprises as an active part an assembly 126, which comprises a source 104 and a detector 106. The source 104 in this case is an X-ray source and the detector 106 an X-ray detector. The assembly 126 is rotatably mounted to a body or housing 108 (not explicitly shown in FIG. 1) such that the assembly 126 is rotated while performing a CBCT scan of a patient. The assembly 126 is hence used for performing a diagnosis operation.

The CBCT machine 102 comprises a forehead support 120 and temple supports 122 for aligning the patient's head anatomy to a desired position prior to initiating the diagnosis operation. However, it may happen that the patient voluntarily or involuntarily moves their head while the diagnosis operation is being performed. In computerized machines, a medical scan is typically generated from one or more scan datasets. Thus, in this example, tomographic images are acquired as one or more scan datasets. Due to head movement of the patient during the medical scan, one or more of the scan datasets may be poor. The present teachings propose an intraoral part 112 to alleviate at least some of the problems associated with head movement.

The diagnosis system 102 thus also comprises the intraoral part 112, which in this example is shown mounted to a grip 124. The grip 124 is attached to a console 110, which in turn is attached to the body or housing 108. The intraoral part 112 is mounted to the grip 124 via an adjusting mechanism 118 which can be used to adjust the height of the intraoral part 112 according to the patient's position. The intraoral part 112 comprises an intraoral portion 114 which is located inside the patient's oral cavity while the medical scan is performed. Optionally, the intraoral part 112 may also comprise an extraoral portion 116 which is located outside of the patient's mouth while the diagnosis operation is performed. It shall be appreciated that the intraoral part 112 is positioned deterministically relative to the active part or assembly 126. The deterministic positioning in this example is achieved by keeping the intraoral part 112 fixedly mounted with respect to the assembly 126 during the diagnosis operation. In some cases, the deterministic positioning may be in the form of a movable connection of the intraoral part 112. In such cases, a change in position of the intraoral part 112 may be measured and the corresponding active part adapted accordingly. The applicant has found this especially beneficial treatment applications where the active part used for treatment can be positioned correctively by taking into account any movement in the intraoral part itself.

The intraoral part 112 is used for monitoring one or more intraoral features of the patient during the diagnosis operation. The intraoral part 112 may comprise and/or it may be operatively coupled to one or more sensors. The one or more sensors are used for monitoring one or more intraoral features of the patient. The one or more sensors generate intraoral data which include information of said features during acquisition of the scan datasets or tomographic images. More preferably for CBCT applications, the sensors are located external of the intraoral part 112 such that objects which may cause artifacts can be avoided in the X-ray path. In such cases, the intraoral part 112 may be operatively coupled to the one or more sensors via an optical path. The optical path may comprise refractive and/or reflective elements such as any one or more of: prisms, mirrors, optical channels etc. The optical path transmits optical information related to the intraoral features to the sensors which in turn transform the optical information to intraoral data which can be analyzed via a computer processor. Similarly, the computer processor may preferably also lie outside of the intraoral part 112. Similarly, the intraoral part 112 may be operatively coupled to one or more light sources which may be located in the intraoral part 112 and/or external to it.

Nevertheless, the intraoral part 112 is operatively connected to one or more computing units (not explicitly shown in FIG. 1). As it was discussed, dependent upon the diagnosis system, any of the computing units may be located in the intraoral part and/or external to it. For example, at least one of the computing units may be located in the console 110 or the housing 108. In some cases, at least some of the computing units may be part of a cloud computing service.

It is determined via any of the computing units those of the scan datasets during acquisition of which a movement related to the intraoral features is detected. Based on the determination, the medical scan is constructed from the acquired scan datasets and/or it may be corrected. The quality of the medical scan can thus be improved.

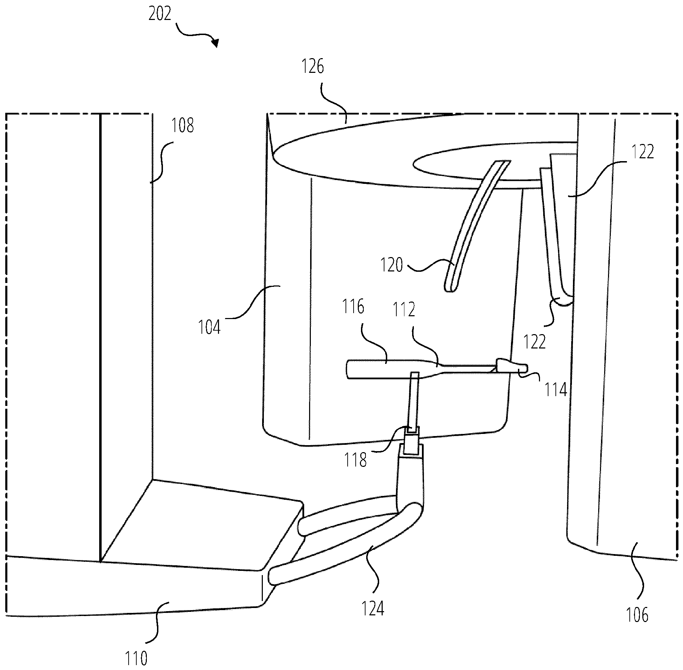

FIG. 2 illustrates another arrangement 202 of a similar CBCT machine 102 as shown in FIG. 1. In contrast to the diagnosis system 102, the another arrangement 202 has the intraoral part 112 mounted horizontally. Other parts and method are similar as were discussed in FIG. 1.

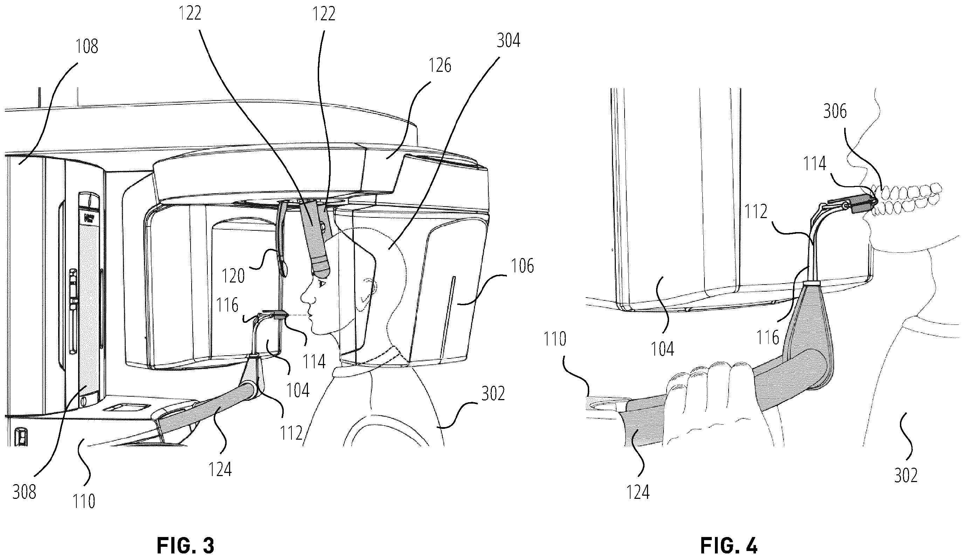

FIG. 3 shows a more detailed diagram of the CBCT machine 102. A patient 302 is also shown positioned in the patient area of the CBCT machine 102. Prior to initiating the diagnosis operation, the patient 302 would move their head anatomy 304 forward such that the patient's forehead touches the forehead support 120 and the temple supports 122 are positioned properly. In some cases, the CBCT machine 102 also comprises a mirror 308 for aiding in alignment of the patient's head anatomy 304 e.g., with respect to the forehead support 120. During the diagnosis operation the patient 302 may hold the grip 124 tightly to help keeping their head as stationary as possible with respect to the CBCT machine 102.

In FIG. 3 and FIG. 4 the intraoral portion 114 is shown as a bite block which the patient 302 can bite prior to starting the diagnosis operation. The patient 302 is then asked to hold the bite position while the diagnosis operation is performed. This can help reducing movement in the head anatomy 304. However, movements may still occur if the bite is not tight and/or if the head tilts in either direction. By monitoring one or more intraoral features in the intraoral data generated via the intraoral part 112, the head movements can be corrected and/or compensated for in the medical scan.

FIG. 4 illustrates a zoomed in view of the patient 302 while the intraoral portion 114 in positioned in their oral cavity. One or more intraoral features 306, shown here as teeth may be monitored via the intraoral part 112 as discussed previously. Although adjusting mechanism 118 is not discussed explicitly in FIG. 3 and FIG. 4, a similar mechanism may be present for adjusting the intraoral part 112 according to the patient 302.

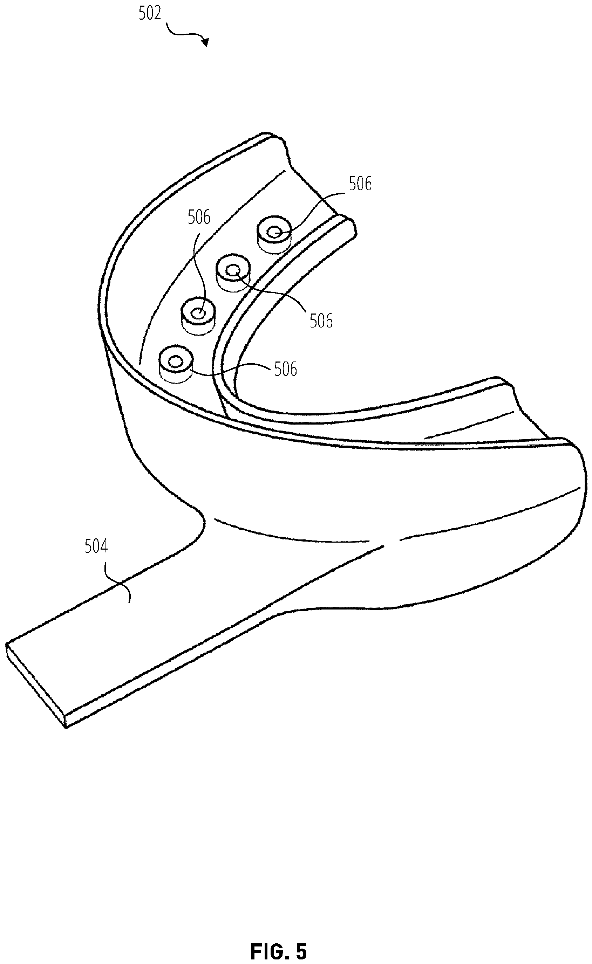

FIG. 5 shows an example of a bite block 502 which may be the intraoral portion 114 in some cases. The bite block 502 comprises one or more optical elements 506 which are used for capturing optical information related to one or more intraoral feature 306. The optical elements 506 may comprise sensors and/or light sources. In some cases, any or all of the optical elements 506 may be a purely refractive and/or reflective elements such as any one or more of: lenses, prisms, mirrors, or optical channels. In such case, the optical information from the optical elements 506 may be transmitted via one or more channels 504. In case the optical elements 506 and/or the bite block 502 comprises electronics, the channel 504 may transmit electrical signals. The advantage of having a purely optical or electronics-free bite block 502 can be that the bite block 502 may be made disposable and/or it may be easier to sanitize, e.g., by high-temperature treatment such as autoclave.

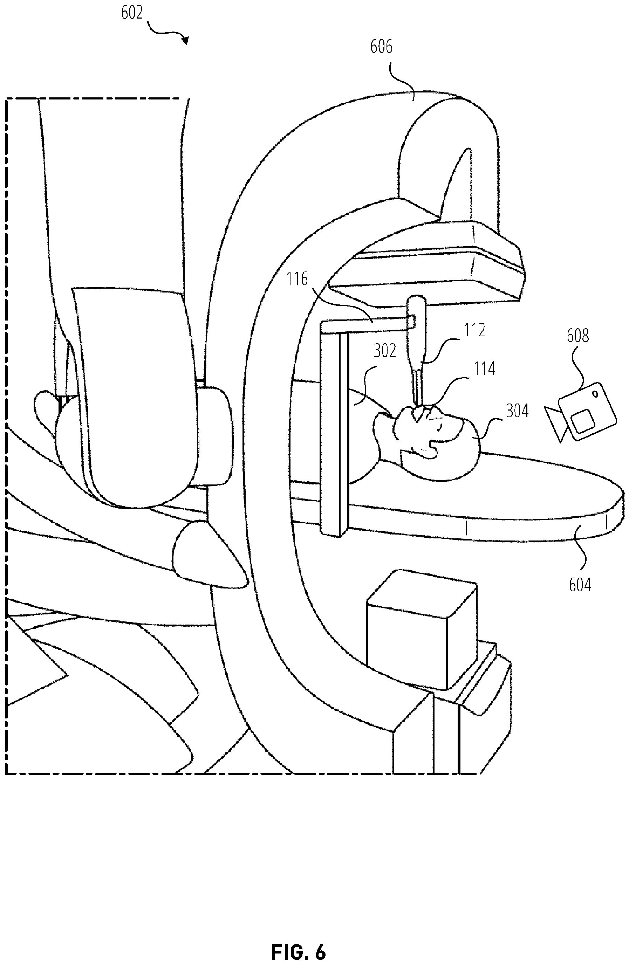

FIG. 6 illustrates an alternate setup 602 which may relate to just a diagnosis system, just a treatment system, or a combination of both. A patient 302 is shown lying on a bed or treatment table 604 which is positioned at a diagnosis/treatment system 606. The diagnosis/treatment system 606 comprises an active part, which is here shown as a radiation source 608. For applying the treatment at the correct location, it may be important to align the patient 302 with respect to the radiation source 608. When the treatment is done in multiple sessions which are spread times apart, it may be difficult to align the patient 302 in a subsequent session to a similar position as in a previous session.

As it was discussed, an intraoral part 112 is provided, of which intraoral portion 114 is positioned in the mouth of the patient 302 for monitoring their intraoral features and providing intraoral data. By monitoring the intraoral features, it is determined, from the intraoral data, if a movement related to the intraoral features is detected. In such cases, where diagnosis and/or treatment need to be performed in multiple sessions, the intraoral data may also comprise previous intraoral data. The previous intraoral data may for example be in the form of reference position data which are used for aligning the intraoral features 306 at the same or similar position relative to the intraoral part 112, as in the previous session. This can result in more comparable diagnosis and/or more effective and targeted treatment. By monitoring the intraoral features 306 between sessions, the patient 302 can be positioned with respect to a desired position so that the treatment can be applied where the disease (e.g., tumor) is located. By monitoring the intraoral features 306 the patient can be more correctly positioned between several treatment sessions. Even if the patient 302 is repositioned by a movement of the treatment table 604, by deterministically positioning the active part or the radiation source 608 with respect to the intraoral part 112, an aligned state can still be achieved.

Additionally, as it was discussed previously, the treatment and/or diagnosis is may be adapted in response to the movement during the session itself. For doing so, for example an active part which is used for performing the treatment and/or diagnosis can be manipulated in response to the movement. Alternatively, or in addition, the medical scan may be corrected or built accordingly as it was explained previously.

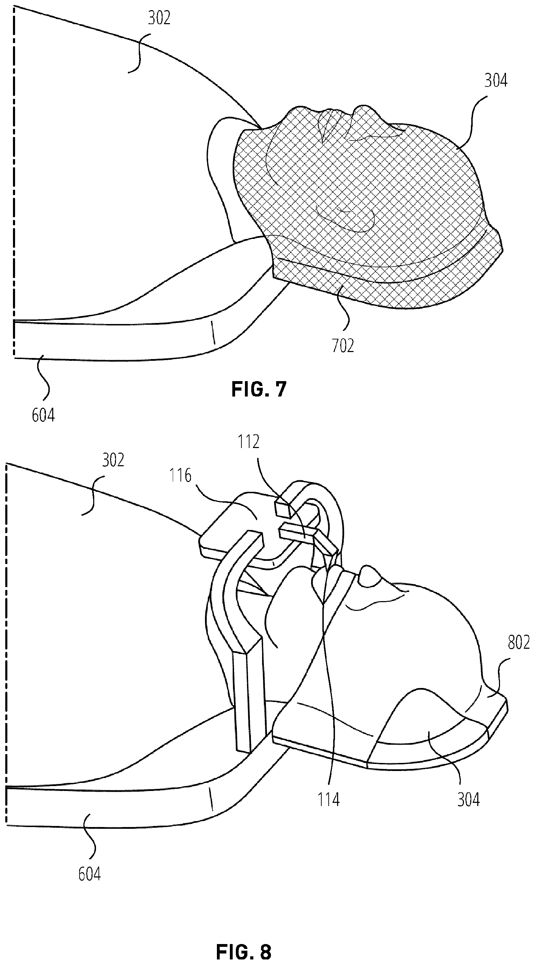

FIG. 7 illustrates an example of an immobilizing means in the form of a net 702, which is placed around the head anatomy 304 of the patient 302. The net 702 is held tightened to a fixed structure such as the treatment table 604. The purpose of the net 702 is to prevent the patient 302 from moving their head, for example, during a treatment. Such immobilizing means can cause discomfort or pain to the patient 302. To reduce the discomfort the net 702 may be loosened, but then the immobilization may be reduced and hence head movement may occur which may interfere in the treatment and/or diagnosis.

FIG. 8 illustrates an example where, in a scenario similar to that shown in FIG. 7, an intraoral part 112 is used to track movement in the head anatomy 304 of the patient 302. The intraoral part 112 is shown fixedly attached to the treatment table 604, but alternatively it can also be movably but deterministically attached as explained previously. An intraoral portion 114 is inserted in the mouth of the patient 302 for monitoring one or more intraoral features. In response to the movement detected from the intraoral data, the treatment and/or diagnosis may be adapted. An immobilizing means 802, e.g., a thermoplastic mask, is shown also in FIG. 8, but at least in some cases, the immobilizing means 802 may be eliminated as the detection of movement can allow adapting the treatment rather than the necessity to hold the head anatomy 304 in a fixed position. In other cases, the immobilizing means 802, if still required, may be loosened to improve comfort for the patient 302.

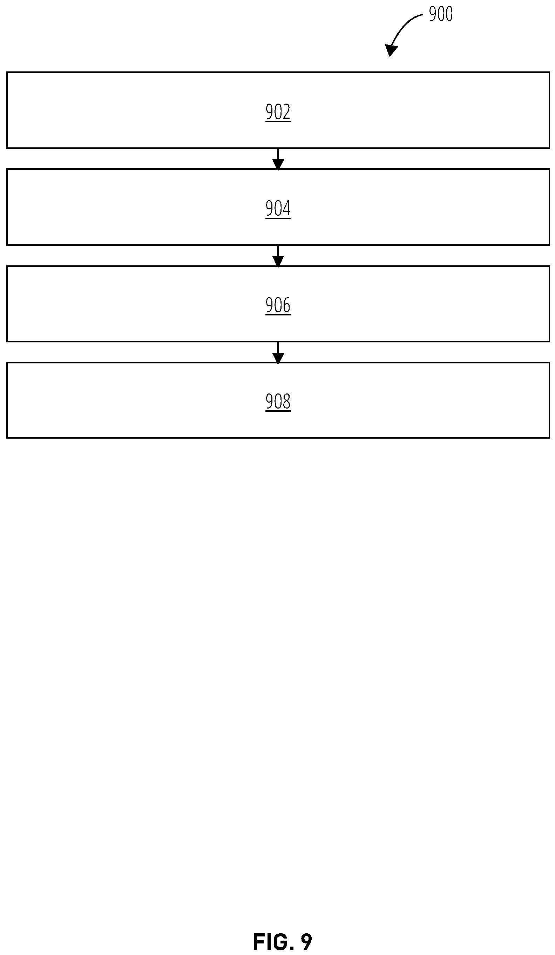

FIG. 9 shows a flowchart 900 illustrating a method aspect of the present teachings, which may be implemented as a computer routine executable by one or more computing units. The method aspects shall also be clear to the skilled persons from the system aspects discussed in previous figures.

For example, for improving a medical scan of head anatomy of a patient, in block 902, it is provided a plurality of scan datasets related to the head anatomy 304, wherein at least some of the scan datasets are usable for obtaining the medical scan. In block 904, it is provided intraoral data related to one or more intraoral features 306 of the patient, which intraoral data having been captured during acquisition of the scan datasets. In block 906, it is determined, using the intraoral data, those parts of the scan datasets during acquisition of which a movement related to the intraoral features 306 is detected. In block 908, in response to the determination, the medical scan is constructed and/or corrected from the scan datasets.

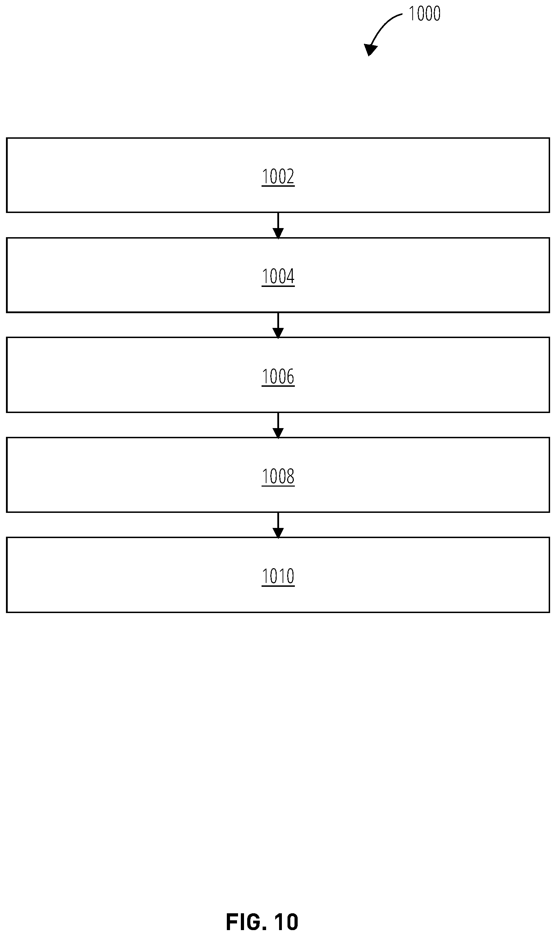

FIG. 10 shows another flowchart 1000 illustrating another, but similar, method aspect of the present teachings, which may be implemented as a computer routine.

For example, for improving a medical scan of head anatomy 304 of a patient 302, in block 1002, it is provided an intraoral part 112 deterministically positioned relative to the acquisition part, e.g., the assembly 126. In block 1004, it is acquired, via the acquisition part, a plurality of scan datasets of the head anatomy 304. In block 1006, it is captured during acquisition of the scan datasets, via the intraoral part, intraoral data related to one or more intraoral features 306. In block 1008, it is determined, using the intraoral data, those of the scan datasets during acquisition of which a movement related to the intraoral feature 306 is detected. In block 1010, the medical scan is constructed and/or corrected based upon the determination.

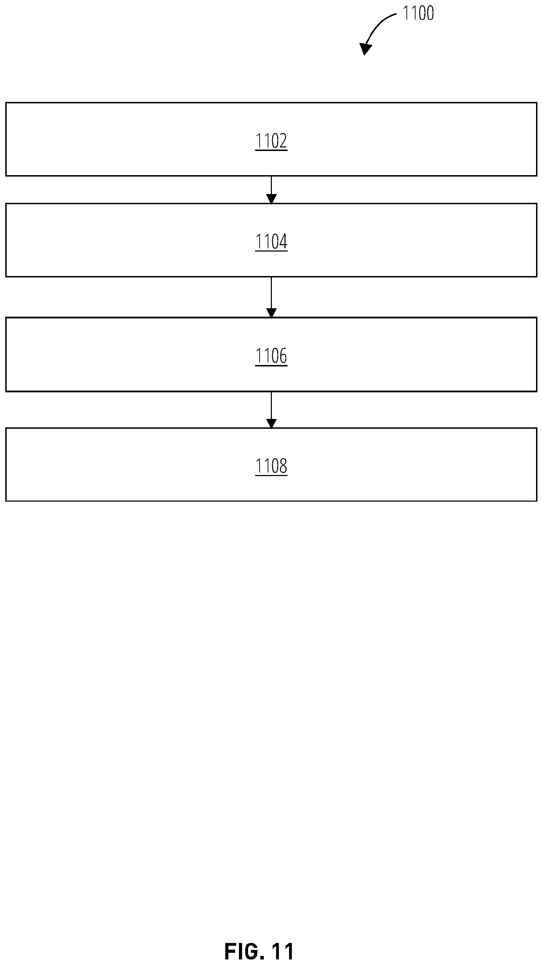

FIG. 11 shows yet another flowchart 1100 illustrating yet another, but similar, method aspect of the present teachings, which may be implemented as a computer routine.

For example, for improving a computer tomography scan of cranial and/or mandibular anatomy or head anatomy 304 of a patient 302, in block 1102, it is provided a plurality of tomographic images of the cranial and/or mandibular anatomy, wherein at least some of the tomographic images are usable for obtaining the computer tomography scan. In block 1104, it is provided intraoral data related to one or more intraoral features 306, which intraoral data having been captured during acquisition of the tomographic images. In block 1106, it is determined, using the intraoral data, those of the tomographic images during acquisition of which a movement related to the intraoral features 306 is detected. In block 1108, the computer tomography scan is constructed and/or corrected based upon the determination.

The method steps may be performed in the order as shown listed in the examples or aspects. It should be noted, however, that under specific circumstances a different order may also be possible. Further, it is also possible to perform one or more of the method steps once or repeatedly. These steps may be repeated at regular or irregular time periods. Further, it is possible to perform two or more of the method steps simultaneously or in a timely overlapping fashion, specifically when some or more of the method steps are performed repeatedly. The method may comprise further steps which are not listed.

The word "comprising" does not exclude other elements or steps, and the indefinite article "a" or "an" does not exclude a plurality. A single processing means, processor or controller or other similar unit may fulfill the functions of several items recited in the claims. The mere fact that certain measures are recited in mutually different dependent claims does not indicate that a combination of these measures cannot be used to advantage. Any different signs in the claim should not be construed as limiting the scope.

Further, it should be noted that in the present disclosure, the terms "at least one", "one or more" or similar expressions indicating that a feature or element may be present once or more than once typically may have been used only once when introducing the respective feature or element. Thus, in some cases unless specifically stated otherwise, when referring to the respective feature or element, the expressions "at least one" or "one or more" may not have been repeated, notwithstanding the fact that the respective feature or element may be present once or more than once.

Further, the terms "preferably", "more preferably", "particularly", "more particularly", "specifically", "more specifically" or similar terms are used in conjunction with optional features, without restricting alternative possibilities. Thus, any features introduced by these terms are optional features and are not intended to restrict the scope of the claims in any way. The present teachings may, as those skilled in the art will recognize, be performed by using alternative features. Similarly, the features introduced by "according to an aspect" or similar expressions are intended to be optional features, without any restriction regarding alternatives to the present teachings, without any restrictions regarding the scope of the present teachings and without any restriction regarding the possibility of combining the features introduced in such way with other optional or non-optional features of the present teachings.

Unless otherwise defined, all technical and scientific terms used herein have the same meaning as commonly understood by one of ordinary skill in the art to which the present teachings belong.

Various examples have been disclosed above for a method, a system, a device, a use, software program, and a computing unit comprising the computer program code for carrying out the methods herein disclosed. For example, it has been disclosed a method for improving a medical scan of head anatomy of a patient comprising: providing a plurality of scan datasets related to the head anatomy; providing intraoral data related to one or more intraoral features of the patient; determining, using the intraoral data, those parts of the scan datasets during acquisition of which a movement related to the intraoral features is detected; constructing and/or correcting the medical scan, from the scan datasets, in response to the determination. The present teachings also relate to a method of improving a CT scan, a system, a device, and a storage medium. Those skilled in the art will understand however that changes and modifications may be made to those examples without departing from the spirit and scope of the accompanying claims and their equivalence. It will further be appreciated that aspects from the method and product embodiments discussed herein may be freely combined.

Any headings utilized within the description are for convenience only and have no legal or limiting effect.

Summarizing and without excluding further possible embodiments, certain example embodiments of the present teachings are summarized in the following clauses:
Clause 1. A computer-implemented method for improving a medical scan of head anatomy of a patient, which method comprises:
   providing a plurality of scan datasets related to the head anatomy, wherein at least some of the scan datasets are usable for obtaining the medical scan;
   providing intraoral data related to one or more intraoral features of the patient, which intraoral data having been captured during acquisition of the scan datasets;
   determining, using the intraoral data, those parts of the scan datasets during acquisition of which a movement related to the intraoral features is detected;
   constructing and/or correcting the medical scan, from the scan datasets, in response to the determination.
Clause 2. The method of clause 1, wherein the constructing and/or correcting of the medical scan involves performing any one or more operations for reducing the effect of the movement from the medical scan, more particularly the operations including any one or more of: deleting, replacing, averaging, compensating, recalculating, extrapolating, or masking, data of the parts of the scan datasets.
Clause 3. The method of clause 1 or clause 2, wherein the correcting of the medical scan involves compensating for the movement which occurred in the corresponding one or more scan datasets.
Clause 4. The method of any of the above clauses, wherein the correcting of the medical scan involves transforming coordinates from the intraoral data to coordinates related to the scan datasets.
Clause 5. The method of any of the above clauses, wherein the method also comprises:
   applying a relative coordinate transformation determined from the intraoral data to a coordinate system of the medical scan.
Clause 6. The method of any of the above clauses, wherein the method also comprises:
   determining one or more reference intraoral positions with respect to one or more of the intraoral features.
Clause 7. The method of clause 6, wherein the method also comprises:
   determining one or more movement values by measuring displacement of the one or more of the intraoral features from their respective reference intraoral positions.
Clause 8. A computer-implemented method for improving a medical scan of head anatomy of a patient, which method comprises:
   providing an intraoral part deterministically positioned relative to an acquisition part;
   acquiring, via the acquisition part, a plurality of scan datasets of the head anatomy;
   capturing during acquisition of the scan datasets, via the intraoral part, intraoral data related to one or more intraoral features;
   determining, using the intraoral data, those of the scan datasets during acquisition of which a movement related to the intraoral features is detected;
   constructing and/or correcting the medical scan based upon the determination.
Clause 9. A computer-implemented method for improving a computer tomography scan of cranial and/or mandibular anatomy of a patient, which method comprises:
   providing a plurality of tomographic images of the cranial and/or mandibular anatomy, wherein at least some of the tomographic images are usable for obtaining the computer tomography scan;
   providing intraoral data related to one or more intraoral features, which intraoral data having been captured during acquisition of the tomographic images;
   determining, using the intraoral data, those of the tomographic images during acquisition of which a movement related to the intraoral features is detected;
   constructing and/or correcting the computer tomography scan based upon the determination.
Clause 10. A computer software product, or a non-transitory computer-readable storage medium storing the program, comprising instructions which when executed by a suitable computing unit cause the computing unit to perform the steps of any of the above method clauses.
Clause 11. A system, wherein the system is configured to perform the steps of any of the above method clauses.
Clause 12. A system comprising means to perform the method of any of the above method clauses.
Clause 13. A medical scanning system comprising:
   an active part for performing the medical scan;
   an intraoral part positioned deterministically relative to the active part; the intraoral part comprising: an intraoral portion which is positionable in the oral cavity of the patient prior to acquiring a plurality of scan datasets; and a sensor for monitoring one or more intraoral features of the patient and generating intraoral data including said features during acquisition of the tomographic images,
   a computing unit configured to:
      determine, using the intraoral data, those of the scan datasets during acquisition of which movement related to the intraoral features is detected; and
      construct and/or correct medical scan based upon the determination, wherein the medical scan is built from the scan datasets.
Clause 14. A system comprising:
   an active part for performing treatment and/or diagnosis of a patient;
   an intraoral part deterministically positionable relative to the active part; the intraoral part comprising: an intraoral portion which is positionable in oral cavity of the patient prior to performing the treatment and/or diagnosis; and, a sensor for: monitoring one or more intraoral features of the patient; and, generating intraoral data including said features during the treatment or diagnosis,
   a computing unit configured to:
      determine, from the intraoral data, a movement related to the intraoral features; and
      adapt the treatment and/or diagnosis in response to the movement.
Clause 15. A system comprising:
   an active part for performing treatment and/or diagnosis of a patient;
   an intraoral part deterministically positionable relative to the active part; the intraoral part comprising: an intraoral portion which is positionable in oral cavity of the patient prior to performing the treatment and/or diagnosis; and, a sensor for: monitoring one or more intraoral features of the patient; and, generating intraoral data including said features,
   a computing unit configured to:
      determine, from the intraoral data, a movement and/or position related to the intraoral features; and
      adapt the treatment and/or diagnosis in response to the movement.

## Claims

1. A computer-implemented method for improving a medical scan of head anatomy of a patient, which method comprises:
providing a plurality of scan datasets related to the head anatomy, wherein at least some of the scan datasets are usable for obtaining the medical scan;
providing intraoral data related to one or more intraoral features of the patient, which intraoral data having been captured during acquisition of the scan datasets;
determining, using the intraoral data, those parts of the scan datasets during acquisition of which a movement related to the intraoral features is detected;
constructing and/or correcting the medical scan, from the scan datasets, in response to the determination.

2. The method of claim 1, wherein the constructing and/or correcting of the medical scan involves performing any one or more operations for reducing the effect of the movement from the medical scan, more particularly the operations including any one or more of:
deleting, replacing, averaging, compensating, recalculating, extrapolating, or masking, data of the parts of the scan datasets.

3. The method of claim 1 or claim 2, wherein the correcting of the medical scan involves compensating for the movement which occurred in the corresponding one or more scan datasets.

4. The method of any of the above claims, wherein the correcting of the medical scan involves transforming coordinates from the intraoral data to coordinates related to the scan datasets.

5. The method of any of the above claims, wherein the method also comprises:
applying a relative coordinate transformation determined from the intraoral data to a coordinate system of the medical scan.

6. The method of any of the above claims, wherein the method also comprises:
determining one or more reference intraoral positions with respect to one or more of the intraoral features.

7. The method of claim 6, wherein the method also comprises:
determining one or more movement values by measuring displacement of the one or more of the intraoral features from their respective reference intraoral positions.

8. A computer-implemented method for improving a medical scan of head anatomy of a patient, which method comprises:
providing an intraoral part deterministically positioned relative to an acquisition part;
acquiring, via the acquisition part, a plurality of scan datasets of the head anatomy;
capturing during acquisition of the scan datasets, via the intraoral part, intraoral data related to one or more intraoral features;
determining, using the intraoral data, those of the scan datasets during acquisition of which a movement related to the intraoral features is detected;
constructing and/or correcting the medical scan based upon the determination.

9. A computer-implemented method for improving a computer tomography scan of cranial and/or mandibular anatomy of a patient, which method comprises:
providing a plurality of tomographic images of the cranial and/or mandibular anatomy, wherein at least some of the tomographic images are usable for obtaining the computer tomography scan;
providing intraoral data related to one or more intraoral features, which intraoral data having been captured during acquisition of the tomographic images;
determining, using the intraoral data, those of the tomographic images during acquisition of which a movement related to the intraoral features is detected;
constructing and/or correcting the computer tomography scan based upon the determination.

10. A computer software product, or a non-transitory computer-readable storage medium storing the program, comprising instructions which when executed by a suitable computing unit cause the computing unit to perform the steps of any of the above method claims.

11. A system, wherein the system is configured to perform the steps of any of the above method claims.

12. A system comprising means to perform the method of any of the above method claims.

13. A medical scanning system comprising:
an active part for performing the medical scan;
an intraoral part positioned deterministically relative to the active part; the intraoral part comprising: an intraoral portion which is positionable in the oral cavity of the patient prior to acquiring a plurality of scan datasets; and a sensor for monitoring one or more intraoral features of the patient and generating intraoral data including said features during acquisition of the tomographic images,
a computing unit configured to:
determine, using the intraoral data, those of the scan datasets during acquisition of which movement related to the intraoral features is detected; and
construct and/or correct medical scan based upon the determination.

14. A system comprising:
an active part for performing treatment and/or diagnosis of a patient;
an intraoral part deterministically positionable relative to the active part; the intraoral part comprising: an intraoral portion which is positionable in oral cavity of the patient prior to performing the treatment and/or diagnosis; and, a sensor for: monitoring one or more intraoral features of the patient; and, generating intraoral data including said features during the treatment or diagnosis,
a computing unit configured to:
determine, from the intraoral data, a movement related to the intraoral features; and
adapt the treatment and/or diagnosis in response to the movement.

15. A system comprising:
an active part for performing treatment and/or diagnosis of a patient;
an intraoral part deterministically positionable relative to the active part; the intraoral part comprising: an intraoral portion which is positionable in oral cavity of the patient prior to performing the treatment and/or diagnosis; and, a sensor for: monitoring one or more intraoral features of the patient; and, generating intraoral data including said features,
a computing unit configured to:
determine, from the intraoral data, a movement and/or position related to the intraoral features; and
adapt the treatment and/or diagnosis in response to the movement.
